# DEMANDE DE BREVET EUROPEEN

(11) **EP 1 938 863 A1**
(43) Date de publication de la demande: **02.07.2008**
(21) Numéro de dépôt: 07291560.6
(22) Date de dépôt: 19.12.2007
(51) Int. Cl.: A61N 1/375, H05K 1/11, H05K 1/18, H05K 3/36

(54) **Procédé d'assemblage mécanique et d'interconnexion électrique des éléments fonctionnels d'un dispositif médical implantable actif**

(30) Priorité: 26.12.2006 FR 0611344
(71) Demandeur: ELA MEDICAL, 92541 Montrouge (FR)
(72) Inventeur: Degieux, Stéphane, 92330 Sceaux (FR); Legay, Thierry, 91640 Fontenay Les Briis (FR)
(74) Mandataire: Dupuis-Latour, Dominique

(57) **Abrégé**

Une première étape consiste à préparer un circuit souple d'interconnexion (20) apte à être relié électriquement et mécaniquement à un module de circuit électronique (14), une pile d'alimentation (12) et une série de picots de traversée (16) du dispositif avent leur placement dans un boîtier. Le circuit souple comprend une série de plages (34) de liaison à des métallisations homologues (46) du substrat. L'assemblage mécanique et la liaison électrique de ces plages (34) aux métallisations (46) est exécuté sans utilisation d'un flux d'activation ni apport de brasure fusible, et comporte le report d'un matériau conducteur anisotrope intercalaire (38) entre les plages (34) et les métallisations (46) puis la polymérisation de ce matériau, ces opérations de report et de polymérisation étant exécutées à pression, température et durées contrôlées.

## Description

L'invention concerne la technologie de fabrication des dispositifs médicaux implantables actifs, notamment la technologie d'assemblage des différents éléments fonctionnels constituant ces dispositifs et destinés à être placés dans un boîtier commun après avoir été interconnectés.

L'invention sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" (DMIA) tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs médicaux implantables actifs (ci-après "dispositifs" ou "dispositifs implantables") se présentent sous la forme d'un boîtier contenant une pile d'alimentation et une plaquette de circuit hybride (ci-après "circuit électronique") supportant et interconnectant l'ensemble des composants, actifs et passifs permettant le recueil et l'analyse des signaux, la génération des impulsions électriques, la mémorisation d'informations de suivi médical, le pilotage des diverses fonctions du dispositif, etc.

La fabrication du dispositif comporte généralement des étapes consistant, dans un premier temps, à interconnecter entre eux les différents éléments fonctionnels, à savoir : la pile, le ou les circuits électroniques ainsi qu'une série de picots de traversée destinés à être reliés ultérieurement à une borne correspondante d'une tête de connecteur. L'étape suivante consiste à placer cet ensemble à l'intérieur du boîtier, puis à adjoindre à ce dernier une tête de connecteur qui servira à assurer la liaison électrique et mécanique du boîtier à des éléments externes, notamment à des sondes de recueil de signaux et/ou d'application d'impulsions.

L'interconnexion des éléments fonctionnels est généralement réalisée au moyen d'un circuit souple portant des pistes conductrices reliées électriquement et mécaniquement à ces éléments fonctionnels avant que ceux-ci ne soient placés dans le boîtier.

Le US 6 245 092 B1 divulgue un stimulateur cardiaque réalisé selon un telle technologie.

Les pistes conductrices se présentent sous la forme de conducteurs imprimés définissant à leur extrémité une plage ou *pad* destinée à être reliée à une métallisation homologue de l'un des éléments fonctionnels.

Cette liaison est réalisée par brasure, technique éprouvée donnant d'excellents résultats en termes de performances électriques et mécaniques et de fiabilité sur le long terme.

L'emploi d'une brasure, typiquement une brasure étain-plomb, implique toutefois l'application d'un flux d'activation de brasure, avec ensuite un nettoyage soigneux du substrat pour éliminer toute trace de flux. Jusqu'à présent, ce nettoyage était opéré de façon très efficace avec des produits à base de CFC, mais l'interdiction de ces produits rend aujourd'hui cette étape de nettoyage plus délicate, avec un risque accru de formation de dendrites de métal résultant de la migration de l'alliage étain-plomb dans certaines régions du substrat qui n'auraient pas été parfaitement préparées.

Au surplus, l'interdiction prochaine des alliages contenant du plomb pour les brasures exacerbe ces difficultés, notamment dans le cas des DMIA. En effet, compte tenu de la place réduite disponible à l'intérieur du boîtier, la miniaturisation poussée de ces dispositifs impose de réaliser des plages de liaison avec des pas très réduits (typiquement 1,47 mm) et des intervalles extrêmement étroits entre ces plages métalliques (l'intervalle entre plages adjacentes est typiquement de l'ordre de 250 µm). Ces problèmes résultant de la compacité extrême des circuits sont encore aggravés par la réduction de la dimension des boîtiers recevant ces circuits, dont le volume a pu être aujourd'hui réduit de 10 cm³ jusqu'à 8,5 cm³.

Le US 6 245 092 B1 précité suggère incidemment, pour éviter le plomb de la brasure, de remplacer l'étape de brasage par une étape de collage.

Le recours au collage - technologie en elle-même bien connue et maîtrisée - pour l'établissement des liaisons électriques aux composants n'est cependant pas dépourvu d'inconvénients, bien au contraire.

En bref, cette technologie consiste essentiellement à remplacer la brasure par une goutte de colle conductrice (colle à l'argent), déposée sur chacune des plages de contact du circuit imprimé souple.

Cette technologie implique donc le dépôt individuel d'une goutte sur chacune des plages de contact, et de ce fait n'est pas appropriée à un traitement collectif, à la différence de la brasure (brasure à la vague par exemple) ce qui complique et renchérit le procédé.

De plus, pour éviter les risques de court-circuit entre deux plages voisines, il est vivement conseillé, si celles-ci sont proches, de déposer une goutte isolante dans l'intervalle séparant les deux gouttes conductrices déposées sur ces plages. Un tel procédé de liaison par collage est d'autant plus délicat à mettre en oeuvre que les dimensions des circuits sont réduites (pas et intervalles des plages), ce qui le rend impropre à des applications où la miniaturisation poussée est une contrainte essentielle. En outre, tant que la colle n'est pas réticulée, il subsiste toujours un risque de migration des particules d'argent en suspension dans les gouttes conductrices, vers la goutte non-conductrice intercalaire.

L'un des buts de la présente invention est de proposer un procédé d'assemblage mécanique et de liaison électrique des plages du circuit souple d'interconnexion aux métallisations homologues de l'élément fonctionnel d'un DMIA, procédé qui évite le recours à des techniques de brasure ou de collage, dont la mise en oeuvre bute aujourd'hui sur des contraintes difficilement contournables.

Le but de l'invention est de proposer un tel procédé qui soit collectif, qui ne nécessite ni flux d'activation, ni nettoyage des surfaces à relier, ni matériau comprenant du plomb, et qui réponde aux contraintes particulières des DMIA, telles que :
- mise en oeuvre possible avec des circuits de faible volume, très confinés et présentant un intervalle extrêmement réduit entre les plages de liaison adjacentes ;
- industrialisation possible de la mise en oeuvre, par un procédé collectif et non opérateur-dépendant ;
- technologie permettant des retouches ponctuelles en cas de défaut détecté en cours de fabrication ;
- utilisation possible avec des circuits réalisés sur des substrats diffusant la chaleur, par exemple des substrats céramique ;
- fiabilité très élevée (la durée de vie d'un implant est habituellement de dix ans, et tout défaut de fonctionnement nécessite l'explantation du dispositif, avec une intervention chirurgicale pour le patient) ;
- ainsi que les rigoureuses contraintes mécaniques et électriques de tenue à l'arrachement, conductivité, isolement, ...

Le principe de base de l'invention consiste à utiliser en lieu et place d'une brasure (en particulier étain-plomb) un matériau conducteur anisotrope polymérisable à la chaleur.

Les matériaux de ce type sont en eux-mêmes déjà connus, notamment pour réaliser des interconnexions entre des circuits souples et un substrat en verre, typiquement pour les écrans à cristaux liquides et les cartes de circuit imprimé. Les conditions de mise en oeuvre dans ces applications connues sont toutefois très différentes de celles rencontrées avec les implants actifs, dans la mesure où le degré de confinement des éléments y est bien moindre, et où la mise en oeuvre se fait sur un support (le verre) qui ne diffuse pas la chaleur. De plus, ce type de liaison était, sur la durée, jusqu'à présent considéré moins fiable que les brasures étain-plomb, réputées pour leur excellente tenue au vieillissement donc procurant un degré de confiance suffisant pour les mettre en oeuvre avec des dispositifs implantés.

Pour cette raison, cette technique a jusqu'à présent été cantonnée aux applications multimédia (interconnexion des écrans et afficheurs), de radiographie (écrans de contrôle) et automobile (instruments et témoins de tableau de bord).

Plus précisément, l'invention propose, pour un dispositif médical implantable actif, un procédé d'assemblage mécanique et d'interconnexion électrique d'éléments fonctionnels d'un dispositif médical implantable actif destinés à être placés dans un boîtier commun après avoir été interconnectés. Ces éléments fonctionnels comprennent au moins un module de circuit électronique, une pile d'alimentation et une série de picots de traversée aptes à être reliés à une tête de connecteur adjointe au boîtier.

Le procédé comprend les étapes de (i) préparation d'un circuit souple d'interconnexion apte à être relié électriquement et mécaniquement auxdits éléments fonctionnels respectifs, ce circuit souple comprenant une série de plages de liaison à des métallisations homologues de l'un desdits éléments fonctionnels, et (ii) assemblage mécanique et liaison électrique desdites plages du circuit souple aux métallisations homologues de l'élément fonctionnel,

De façon caractéristique de l'invention, l'étape d'assemblage mécanique et de liaison électrique des plages du circuit souple aux métallisations de l'élément fonctionnel est une étape (i) n'impliquant essentiellement pour sa mise en oeuvre aucun flux d'activation ni apport de brasure fusible, et (ii) comportant le report d'un matériau conducteur anisotrope intercalaire entre lesdites plages et lesdites métallisations respectives, et la polymérisation de ce matériau, ces opérations de report et de polymérisation étant exécutées à pression, température et durées contrôlées.

Le module de circuit électronique peut être un module hybride avec un substrat, notamment en matériau céramique ou époxy, portant des composants électroniques. L'élément fonctionnel portant lesdites métallisations comprend alors ledit module, et les métallisations sont formées sur le substrat de ce module.

De préférence, l'étape d'assemblage mécanique et de liaison électrique des plages du circuit souple aux métallisations de l'élément fonctionnel est exécutée postérieurement à une étape d'assemblage mécanique et de liaison électrique du circuit souple d'interconnexion à la pile d'alimentation et aux picots de traversée.

L'élément fonctionnel portant lesdites métallisations peut comprendre un autre circuit souple d'interconnexion, et/ou un composant électronique discret sur lequel sont formées lesdites métallisations.

Dans une forme de mise en oeuvre avantageuse, le matériau conducteur anisotrope est un film de type ACF. L'étape d'assemblage mécanique et de liaison électrique des plages du circuit souple aux métallisations de l'élément fonctionnel comprend alors (i) une sous-étape de *pre-tacking* avec collage d'un film ACF sur lesdites plages, ou sur lesdites métallisations, et (ii) une sous-étape de *tacking* avec positionnement desdites plages par rapport auxdites métallisations, puis polymérisation sous pression, cette sous-étape de *tacking* étant exécutée dans des conditions de température, de durée et de pression supérieures aux température, durée et pression de la sous-étape de *pre-tacking.*

En variante, le matériau conducteur anisotrope peut également être une pâte conductrice.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 est une vue en perspective des divers éléments fonctionnels interconnectés par un circuit souple, avant leur mise en place dans le boîtier du générateur.

La figure 2 est une vue en élévation correspondant à la figure 1.

La figure 3 est une vue en plan des éléments fonctionnels après leur mise en place dans le boîtier.

La figure 4 illustre les diverses étapes de *pré-tacking* et de *tacking* propres au procédé d'assemblage de l'invention.

La figure 5 montre, en vue agrandie, l'interface conductrice entre le circuit souple et le substrat du module de circuit électronique.

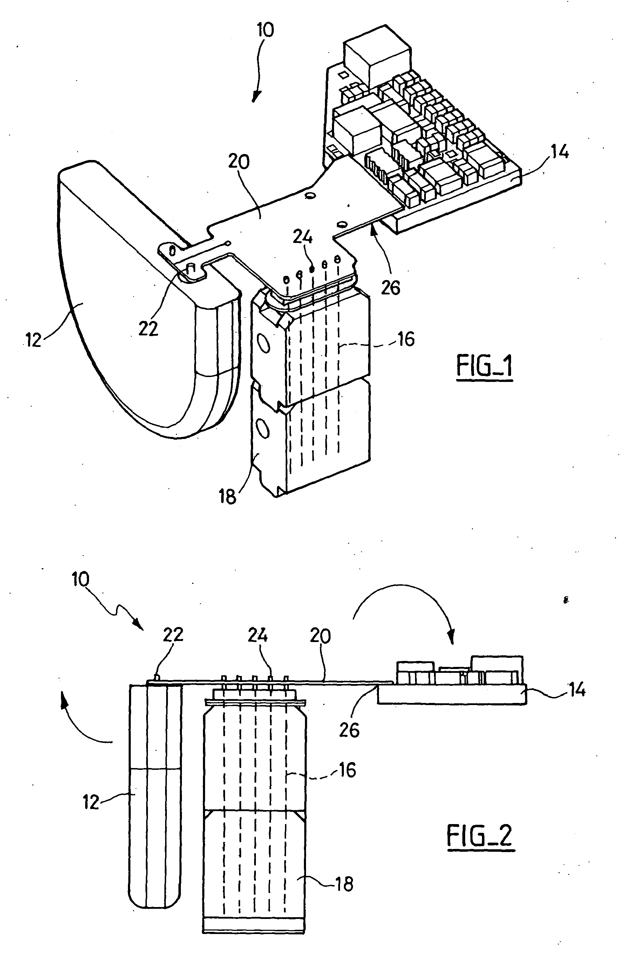

Sur la figure 1, la référence 10 désigne de façon générale l'ensemble des éléments destinés à être placés à l'intérieur du boîtier d'un générateur de stimulateur ou défibrillateur implantable. Ces différents éléments, par la suite désignés de façon générique "éléments fonctionnels", comprennent essentiellement une pile d'alimentation 12, un ou plusieurs modules de circuit électronique 14 et une série de picots de traversée 16, ici représentés munis d'un bouchon 18 pour leur protection jusqu'à la mise en place dans le boîtier.

L'invention peut notamment être appliquée - de façon bien entendu non limitative - à la fabrication des dispositifs implantables commercialisés par ELA Médical, Montrouge, France tels que les appareils *Symphony et ELA Rhapsody.*

Le module de circuit électronique 14 est, dans l'exemple illustré, un module hybride comprenant un substrat unique portant l'ensemble des composants électroniques actifs et passifs du dispositif. Le substrat peut notamment être en un matériau céramique multicouches recevant à l'intérieur de cavités appropriées une ou plusieurs puces de circuit intégré, la cavité étant ensuite obturée par une résine isolante de manière à ménager une surface recevant les divers composants discrets associés. La céramique du substrat peut être enrobée d'une résine époxy ; en variante, le substrat peut également être lui-même constitué d'une résine époxyde.

Les divers éléments fonctionnels sont interconnectés par un circuit souple 20, par exemple un circuit flexible en polyimide, portant en surface diverses pistes métalliques conductrices pour assurer les liaisons électriques entre les bornes 22 de la pile 12, les extrémités 24 des picots 16 et les diverses bornes de liaison du module 14, qui se présentent sous forme d'une série de métallisations de surface. Ces métallisations sont destinées à être reliées électriquement et mécaniquement au circuit souple 20 à l'interface 26 de ce circuit 20 avec le substrat du module 14.

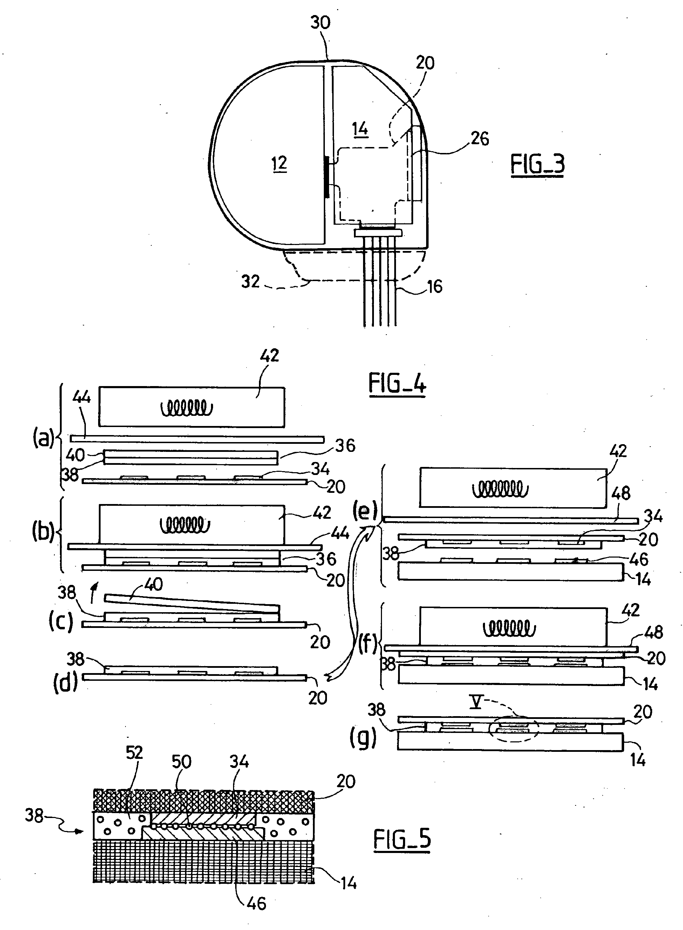

Une fois réalisées les liaisons des éléments fonctionnels au circuit souple d'interconnexion 20, les différents éléments sont amenés sensiblement dans un plan commun, par pliage du circuit souple 20 suivant les flèches illustrées figure 2. La configuration est alors celle illustrée figure 3, avec la pile 12 s'étendant approximativement dans le même plan que le circuit souple 20, et le module 14 retourné à plat contre ce même circuit 20, qui se trouve courbé à 180° au voisinage de l'interface 26. Les picots 16, quant à eux, sont redressés d'un quart de tour par rapport à leur position initiale, pour venir s'étendre sensiblement dans le plan commun des autres éléments positionnés comme indiqué plus haut.

Les différents éléments ainsi configurés pourront alors être placés dans le boîtier 30 du générateur dans une configuration la plus confinée possible, avec un minimum de volume perdu. Dans une étape ultérieure, une tête de connecteur 32 sera rapportée sur le boîtier 30 et les différents organes conducteurs du connecteur seront reliés électriquement aux picots 16.

L'invention concerne plus particulièrement la réalisation de la liaison électrique et mécanique du circuit souple 20 au substrat du module électronique 14 à l'interface 26 de ces deux éléments (dans la mesure où la liaison de la pile 12 et des picots 16 au circuit souple ne pose pas les mêmes difficultés, cette liaison peut être réalisée au niveau des bornes 22 et 24 de manière traditionnelle, par brasure ; mais compte tenu de ses avantages propres que l'on exposera par la suite, l'invention est applicable aussi à la réalisation de ces liaisons).

Il convient de relier électriquement et mécaniquement les extrémités des pistes conductrices du circuit souple 20 à des métallisations homologues formées sur le substrat du module 14 en respectant les multiples contraintes électriques, mécaniques, industrielles et environnementales évoquées dans l'introduction de la présente description.

L'invention propose, de façon caractéristique, de réaliser ces liaisons par un procédé de collage haute température, sous contrainte de pression, du circuit souple au substrat avec interposition d'un film conducteur anisotrope.

Les films conducteurs anisotropes sont en eux-mêmes connus dans les applications évoquées plus haut (multimédia, automobile,...) mais n'avaient jusqu'à présent jamais été envisagés dans le domaine des implants médicaux, compte tenu des conditions bien plus contraignantes de tenue au vieillissement, miniaturisation, ...

Les films conducteurs anisotropes sont connus sous la dénomination de "films ACF" *(Anisotropic Conductive Film)* ou *"Z-Axis Conductive Film".* Il est possible par exemple d'utiliser les films de type ACF commercialisés par la société Hitachi Chemical Co., Ltd. ou tout autre film similaire.

La figure 4 illustre les différentes étapes successives de ce procédé selon l'invention, qui comporte essentiellement deux phases, dites de *"pré-tacking"* (étapes a-d) puis de *"tacking"* (étapes e-g).

La phase de *pré-tacking* consiste à faire adhérer un film ACF sur l'une des deux pièces à assembler, dans l'exemple illustré le circuit souple 20 (mais ce pourrait aussi bien être le substrat 14).

A l'étape a, le circuit souple 20, déjà relié par brasure à la pile 12 et aux picots 16, est positionné sur un outillage approprié.

Un film ACF 36 est positionné, par ce même outillage, au-dessus du circuit souple 20. Ce film comporte, de manière en elle-même connue, une couche conductrice anisotrope (couche ACF) 38, déposée sur une dorsale protectrice 40, par exemple en PET. La couche ACF 38 est tournée vers les plages conductrices 34 du circuit souple 20, et un élément chauffant ou thermode 42 est approché du dos du film ACF 36, avantageusement avec interposition d'un ruban de silicone 46 pour absorber les aspérités et mieux répartir la chaleur.

L'étape suivante b consiste à exécuter une lamination du film ACF 36 sur le circuit souple 20 par thermo-adhésion sous pression. Les conductions de cette étape sont par exemple : température de chauffage 80° C environ, durée d'application de la thermode 3 à 5 secondes, pression d'application 0,5 à 1,5 MPa.

Une fois cette étape de thermocollage effectuée, l'étape suivant c consiste, après retrait de la thermode 42 et du ruban intercalaire 44, à retirer la couche dorsale protectrice 40, de manière à ne laisser subsister sur le circuit souple 20 que l'épaisseur du film ACF proprement dit 38.

La situation à la fin de cette phase de *pré-tacking* est celle illustrée en d figure 4. La phase suivante, de *tacking,* correspond aux étapes illustrées e à g de la figure 4.

A l'étape e, après avoir été retourné recto/verso le circuit souple 20 pourvu du film ACF 38 est placé face au substrat 14, de manière que les plages 34 (recouvertes par le film ACF 38) du circuit souple 20 soient positionnées exactement en vis-à-vis des métallisations 46 présentes en surface du substrat 14.

Comme à l'étape a, la thermode 42 est positionnée au-dessus du tout, avec interposition d'un ruban protecteur de silicone 48 pour absorber les aspérités et mieux répartir la chaleur.

L'étape f consiste à exécuter une lamination de l'ensemble sous pression et haute température de manière à polymériser la résine du film ACF. Les conditions de cette étape sont par exemple : température environ 170° C, durée d'application de la thermode 10 à 15 secondes, pression d'application de 2 à 4 MPa.

La situation finale est celle illustrée en g. La figure 5 montre, en format agrandi, le détail repéré V : à l'interface entre les plages 34 du circuit souple 20 et les métallisations 46 du substrat 14, le film ACF 38 contient des microbilles conductrices 50 noyées dans une pâte 52. La dimension et la concentration de ces microbilles 50 est telle que, en fin de processus, elles permettent d'établir un contact électrique d'excellente qualité entre la plage 34 et la métallisation 46, à l'interface de contact de ces deux éléments. En revanche, en dehors de cette interface, la concentration en microbilles n'est pas suffisante pour que celles-ci viennent en contact mutuel, empêchant ainsi toute liaison électrique entre le circuit souple 20 et le substrat 14 en dehors de l'interface plages/métallisations. On peut ainsi obtenir, typiquement, une impédance de contact plage/métallisation inférieure à 10 Ω et une résistance d'isolement circuit souple/substrat supérieure à 1 GΩ. Ces performances sont conservées même après vieillissement et soumission à des contraintes de chaleur, humidité, etc., ce qui permet de garantir une excellente tenue dans le temps.

Si l'on définit de façon précise et reproductible le profil de température/durée/pression pour les étapes de *pré-tacking* et de *tacking,* il est possible d'industrialiser le processus de manière parfaitement répétitive et reproductible. De plus, en cas de défaut, le produit est "retouchable" (*reworkable),* c'est-à-dire que, de la même manière qu'avec une liaison brasée, le circuit souple dont la connexion est défaillante peut être remplacé par un autre circuit après nettoyage du circuit hybride, cette opération s'effectuant par pelage sur plaque chauffante à une température de l'ordre de 125° C.

En variante, Il est possible de remplacer l'utilisation d'un ruban ACF par une pâte conductrice anisotrope, conditionnée en seringue. Cette pâte pourra notamment être déposée, à température ambiante, sur des sections couvrant les métallisations du substrat ou les plages conductrices du circuit souple, le thermocollage étant ensuite effectué de la même façon que précédemment.

D'autre part, la présente invention n'est pas limitée à la réalisation d'une liaison entre un circuit souple et un substrat de circuit hybride. Elle peut être également utilisée pour relier entre eux deux circuits souples, ou à un composant discret rapporté, tel quel ou sur un *chip carrier.*

## Revendications

1. Un procédé d'assemblage mécanique et d'interconnexion électrique d'éléments fonctionnels (12, 14, 16) d'un dispositif médical implantable actif, destinés à être placés dans un boîtier commun (30) après avoir été interconnectés,
ces éléments fonctionnels comprenant au moins un module de circuit électronique (14), une pile d'alimentation (12) et une série de picots de traversée (16) aptes à être reliés à une tête de connecteur (32) adjointe au boîtier (30),
le procédé comprenant les étapes de :
- préparation d'un circuit souple d'interconnexion (20) apte à être relié électriquement et mécaniquement auxdits éléments fonctionnels respectifs, ce circuit souple comprenant une série de plages (34) de liaison à des métallisations homologues (46) de l'un desdits éléments fonctionnels, et
- assemblage mécanique et liaison électrique desdites plages (34) du circuit souple aux métallisations homologues (46) de l'élément fonctionnel,
ce procédé étant **caractérisé en ce que** l'étape d'assemblage mécanique et de liaison électrique des plages du circuit souple aux métallisations de l'élément fonctionnel est une étape :
- n'impliquant essentiellement pour sa mise en oeuvre aucun flux d'activation ni apport de brasure fusible, et
- comportant le report d'un matériau conducteur anisotrope intercalaire (38) entre lesdites plages (34) et lesdites métallisations (46) respectives, et la polymérisation de ce matériau, ces opérations de report et de polymérisation étant exécutées à pression, température et durées contrôlées.

2. Le procédé de la revendication 1, dans lequel :
- le module de circuit électronique (14) est un module hybride avec un substrat portant des composants électroniques, et
- l'élément fonctionnel portant lesdites métallisations comprend ledit module, les métallisations (46) étant formées sur le substrat de ce module.

3. Le procédé de la revendication 2, dans lequel ledit substrat sur lequel sont formées les métallisations est un substrat en matériau céramique.

4. Le procédé de la revendication 2, dans lequel ledit substrat sur lequel sont formées les métallisations est un substrat en matériau époxy.

5. Le procédé de la revendication 2, dans lequel ladite étape d'assemblage mécanique et de liaison électrique des plages du circuit souple aux métallisations de l'élément fonctionnel est exécutée postérieurement à une étape d'assemblage mécanique et de liaison électrique du circuit souple d'interconnexion à la pile d'alimentation et aux picots de traversée.

6. Le procédé de la revendication 1, dans lequel l'élément fonctionnel portant lesdites métallisations comprend un autre circuit souple d'interconnexion.

7. Le procédé de la revendication 1, dans lequel l'élément fonctionnel portant lesdites métallisations comprend un composant électronique discret, sur lequel sont formées lesdites métallisations.

8. Le procédé de la revendication 1, dans lequel :
- ledit matériau conducteur anisotrope(38) est un film de type ACF, et
- l'étape d'assemblage mécanique et de liaison électrique des plages du circuit souple aux métallisations de l'élément fonctionnel comprend :
. une sous-étape de *pre-tacking* avec collage d'un film ACF sur lesdites plages, ou sur lesdites métallisations, et
. une sous-étape de *tacking* avec positionnement desdites plages par rapport auxdites métallisations, puis polymérisation sous pression, cette sous-étape de *tacking* étant exécutée dans des conditions de température, de durée et de pression supérieures aux température, durée et pression de la sous-étape de *pre-tacking.*

9. Le procédé de la revendication 1, dans lequel ledit matériau conducteur anisotrope est une pâte conductrice anisotrope.
